# EUROPÄISCHE PATENTSCHRIFT

(11) **EP 2 740 468 B1**
(45) Veröffentlichungstag und Bekanntmachung des Hinweises auf die Patenterteilung: **14.09.2016**
(21) Anmeldenummer: 13189543.5
(22) Anmeldetag: 21.10.2013
(51) Int. Cl.: A61K 9/00, A61L 29/16

(54) **Verfahren zur Wirkstofffreisetzung und Wirkstofffreisetzungssysteme**
Method for drug delivery and drug delivery systems
Procédé de libération de principe actif et systèmes de libération de principe actif

(30) Priorität: 07.11.2012 DE 102012021675; 21.12.2012 DE 102012025143
(43) Veröffentlichungstag der Anmeldung: 11.06.2014
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- WO-A1-97/13007
- WO-A2-2007/106557
- LI ET AL: "An electrochemical intraocular drug delivery device", SENSORS AND ACTUATORS A, ELSEVIER SEQUOIA S.A., LAUSANNE, CH, Bd. 143, Nr. 1, 20. März 2008 (2008-03-20) , Seiten 41-48, XP022550305, ISSN: 0924-4247

## Beschreibung

Die Erfindung betrifft eine Vorrichtung zur Freisetzung eines Wirkstoffs sowie ein Verfahren zur Freisetzung eines Wirkstoffs und ein medizinisches Implantat aufweisend eine solche Vorrichtung.

Die Vorrichtung und das Verfahren sind zur aktiven, kontinuierlichen Wirkstofffreisetzung, insbesondere der lokalen Freisetzung von antibiotischen Wirkstoffen bestimmt. Die Wirkstofffreisetzungssysteme können in Form von Spacern, als aktiver Bestandteil von Spacern und auch als Implantat Verwendung finden. Grundsätzlich sind solche Verfahren und Vorrichtungen nicht zur Einnahme vorgesehen, sondern werden mit in den Körper implantierten Vorrichtungen mit Behältnissen für den Wirkstoff umgesetzt. Die Erfindung befasst sich dabei im speziellen mit sogenannten Spacern, die als zwischenzeitlicher Ersatz für Endoprothesen im Körper eingesetzt werden, um eine Infektion des umliegenden Gewebes zu bekämpfen.

Zementierte und nichtzementierte Totalgelenkendoprothesen (TEP) sind heute Stand der Technik in der Orthopädie. Leider gibt es eine geringe Anzahl von Früh- und Spätinfektionen bei TEPs. Infizierte TEPs machen im Allgemeinen eine Wechsel-Operation notwendig. Diese Wechsal-Operationen werden in einzeitige und zweizeitige Operationen unterteilt. Bei der zweizeitigen Wechsel-Operation wird zuerst die infizierte TEP entfernt, das infizierte Knochen- und Weichgewebe debridiert und anschließend ein Spacer als temporärer Platzhalter eingesetzt. Dabei kommen üblicherweise industriell vorgefertigte Spacer zur Anwendung, die ein Antibiotikum enthalten können, oder auch individuelle aus PMMA-Knochenzement gefertigte Spacer, die entweder frei geformt, oder auch unter Verwendung von Gießformen vom Operateur selbst gefertigt werden. Diese Spacer können durch individuelle Dotierung des eingesetzten PMMA-Knochenzements mit Antibiotika, entsprechend dem Antibiogramm der der Infektion zu Grunde liegenden Keime patientenspezifisch gefertigt werden.

Spacer verbleiben meistens mehrere Wochen, vielfach 6 bis 8 Wochen, im Patienten, bis sich die Infektion beruhigt hat. Anschließend wird in einer zweiten Operation der Spacer entfernt, das angrenzende Gewebe nochmals debridiert und dann wird die Revisionsprothese eingesetzt, die zementiert oder auch zementfrei sein kann.

Neben den industriell gefertigten PMMA-Spacern und den individuell gefertigten PMMA-Spacern, die ein Antibiotikum oder auch mehrere Antibiotika enthalten, wurden auch Spacer vorgeschlagen, die selbst keine Antibiotika enthalten. Diese Spacer enthalten Kavitäten, die vom medizinischen Anwender mit antibiotischen Lösungen befüllt werden können.

In der US 2010/042214 A1 werden Spacer offenbart, die wenigstens einen Hohlraum enthalten, der über Kanäle mit der Außenseite der Spacer verbunden ist. Die Wirkstofflösung kann in den Hohlraum eingebracht werden und tritt dann aus den Kanalöffnungen an der Außenseite der Spacer aus.

Ein ähnlicher Spacer wurde in der US 2011/015754 A1 beschrieben, der wenigstens zwei Tanks zur Aufnahme von Wirkstofflösungen besitzt. Diese Tanks haben Öffnungen, durch welche die Wirkstofflösungen in die Umgebung abgegeben werden können.

Mit der US 2010/217401 A1 wurden poröse Schrauben vorgeschlagen, die im Inneren ein Wirkstoffreservoir besitzen, das mit den Poren verbunden ist. Weitere sehr ähnliche Vorrichtungen sind bereits aus den Schriften US 7,794,484 B2, US 7,255,713 B2 und US 7,300,439 B2 bekannt.

Bei diesen befüllbaren Spacern und anderen mit Wirkstofflösungen befüllbaren Wirkstoffträgern treten die in flüssiger gelöster Form vorliegenden Wirkstoffe durch einfache Diffusion aus.

Nachteilig ist hieran, dass es dadurch zu Schwankungen in der Wirkstofffreisetzung kommen kann, dass die Wirkstofffreisetzung zu früh beendet wird und dass es zu einer asymptotischen Abnahme der Freisetzung der Wirkstoffe kommen kann.

Die Aufgabe der Erfindung besteht also darin, die Nachteile des Stands der Technik zu überwinden. Insbesondere soll eine Vorrichtung und ein Verfahren bereitgestellt werden, bei denen Wirkstoffe über einen Zeitraum von bis zu mehreren Wochen kontinuierlich und möglichst auch gleichmäßig freigesetzt werden können.

Die Aufgaben der Erfindung werden gelöst durch eine Vorrichtung zur Freisetzung eines fluiden Wirkstoffs aufweisend wenigstens einen Hohlraum, in dem der fluide Wirkstoff enthalten ist, wobei in der Vorrichtung Wasser und ein Reduktionsmittel enthalten sind, die miteinander in Verbindung stehen oder miteinander in Verbindung bringbar sind, so dass durch eine chemische Reaktion des Wassers mit dem Reduktionsmittel in der Vorrichtung ein Gas entsteht und das Gas derart mit dem fluiden Wirkstoff in Wirkverbindung steht, das der entstehende Gasdruck den fluiden Wirkstoff aus zumindest einer Öffnung des Hohlraums aus der Vorrichtung herausdrückt, wobei der Hohlraum zumindest bereichsweise durch eine Mehrzahl von zumindest bereichsweise parallelen kapillarförmigen Röhren in der Vorrichtung gebildet ist, die sich auf einer Seite bis zu einer Öffnung in der Oberfläche der Vorrichtung erstrecken.

Bevorzugt ist dabei vorgesehen, dass das entstehende Gas Wasserstoff ist.

Gemäß einer Weiterbildung der Erfindung kann vorgesehen sein, dass das Wasser für die chemische Reaktion ein Teil einer flüssigen wässrigen Lösung ist, die den fluiden Wirkstoff bildet, wobei bevorzugt der fluide Wirkstoff eine wässrige Lösung aufweisend zumindest eine pharmazeutisch aktive Substanz ist, besonders bevorzugt eine wässrige Lösung aufweisend zumindest ein Antibiotikum.

Dies macht den Aufbau besonders einfach und kostengünstig umzusetzen, da der fluide Wirkstoff auch gleichzeitig das Treibmittel (das Gas) zum Austreiben des fluiden Wirkstoffs aus der Vorrichtung bereithält.

Ferner kann vorgesehen sein, dass das Reduktionsmittel eine metallische Oberfläche ist, wobei bevorzugt die metallische Oberfläche ein negatives Standardpotential aufweist, besonders bevorzugt die metallische Oberfläche Magnesium, Eisen oder Legierungen aufweisend Magnesium und/oder Eisen beinhaltet oder daraus besteht.

Solche Oberflächen korrodieren langsam, so dass eine gleichmäßige und langfristige Freisetzung des Wirkstoffs mit dem Aufbau sichergestellt werden kann.

Dabei kann vorgesehen sein, dass das Reduktionsmittel Magnesium und ein Edelmetall ist, die eine elektrochemische Zelle bilden, oder das Reduktionsmittel eine Mischung eines schnell korrodierenden Metalls und eines langsam korrodierenden Metalls ist.

Mit dieser Variante kann die Freisetzung des Gases besonders gut und auch langfristig genau eingestellt werden.

Mit einer besonders bevorzugten Weiterbildung der Erfindung kann vorgesehen sein, dass mindestens eine solche Masse an Reduktionsmittel und/oder an Wasser in der Vorrichtung angeordnet ist, dass eine solche Menge an Gas entsteht, die zumindest 30% des Volumens des Hohlraums einnimmt, bevorzugt zumindest 50% des Volumens des Hohlraums, besonders bevorzugt zumindest 80% des Volumens des Hohlraums.

Durch diese Mengen wird sichergestellt, dass ein ausreichender Teil des Wirkstoffs aus der Vorrichtung ausgetrieben wird. Ganz besonders bevorzugt ist vorgesehen, dass eine solche Masse an Reduktionsmittel in der Vorrichtung angeordnet ist, dass eine solche Menge an Gas entsteht, die 70% bis 90% des Volumens des Hohlraums entspricht. Dann ist genau die richtige Menge an Reduktionsmittel vorhanden, so dass auch bei einem längeren Verbleib der Vorrichtung im Körper nicht unnötig viel Gas erzeugt wird, das vom Körper resorbiert werden muss.

Dieser Aufbau ist sehr einfach und sehr sicher. Durch die kapillarförmigen Röhren wird sichergestellt, dass die Oberflächenspannung einer enthaltenen wässrigen Lösung dafür sorgt, dass zunächst die wässrige Lösung aus den kapillarförmigen Röhren ausgetrieben wird, da das Gas nicht durch die Kapillaren an der wässrigen Lösung vorbei gedrückt werden kann. Es ist also erfindungsgemäß besonders bevorzugt vorgesehen, dass die kapillarförmigen Röhren Kapillaren sind. Vorzugsweise haben die Kapillaren einen Durchmesser von weniger als 500 µm, besonders bevorzugt von weniger als 200 µm.

Dabei kann vorgesehen sein, dass das Reduktionsmittel an dem zu der jeweiligen Öffnung gegenüberliegenden Ende der kapillarförmigen Röhren angeordnet ist, wobei das Reduktionsmittel in dem Hohlraum angeordnet ist und nur über die Öffnung der Röhre mit der Oberfläche der Vorrichtung verbunden ist, wobei vorzugweise der fluide Wirkstoff als wässrige Lösung mit dem Reduktionsmittel in Kontakt steht.

Das Gas entsteht so auf der gewünschten Seite der kapillarförmigen Röhren. Zudem können die kapillarförmigen Röhren vorzugsweise an der von der Öffnung nach außen abgewandten Seite der Röhren mit dem Reduktionsmittel beschichtet sein.

Gemäß einer alternativen, besonders bevorzugten Ausführungsform kann vorgesehen sein, dass in dem Hohlraum ein in Richtung der zumindest einen Öffnung des Hohlraums verschiebbarer Kolben angeordnet ist und der Kolben den Hohlraum in einen ersten Teil, der die zumindest eine Öffnung aufweist, und einen zweiten geschlossenen oder verschließbaren Teil unterteilt, wobei in dem ersten Teil der fluide Wirkstoff angeordnet ist und in dem zweiten Teil das Reduktionsmittel und Wasser angeordnet sind, so dass der entstehende Gasdruck in dem geschlossenen, zweiten Teil des Hohlraums den Kolben in Richtung der zumindest einen Öffnung treibt und dadurch den fluiden Wirkstoff aus dem ersten Teil des Hohlraums durch die zumindest eine Öffnung aus der Vorrichtung herausdrückt.

Durch diesen Aufbau kann auch eine größere Menge des Wirkstoffs auf dem gleichen Raum langsam freigesetzt werden, als dies bei der Verwendung mit Kapillaren möglich wäre, da hier der Hohlraum als Nutzraum im Vergleich zu den äußeren Abmessungen der Vorrichtung wesentlich größer ist.

Dabei kann vorgesehen sein, dass in der Innenwand des Hohlraums ein Überströmkanal angeordnet ist, der in einer Ausgangsstellung des Kolbens den ersten Teil und den zweiten Teil des Hohlraums verbindet und der zweite Teil des Hohlraums eine Verschlussöffnung aufweist, die durch einen Verschluss verschließbar oder geschlossen ist, so dass der Hohlraum in beiden Teilen mit der gleichen wässrigen Lösung, die den fluiden Wirkstoff bildet und das flüssige Wasser für die chemische Reaktion bereitstellt, durch die Verschlussöffnung befüllbar ist, wobei der Verschluss derart aufgebaut ist, dass er beim Verschließen der Verschlussöffnung den Kolben in dem Hohlraum in Richtung der zumindest einen Öffnung an dem Überströmkanal vorbei schiebt, so dass der Überströmkanal den ersten und den zweiten Teil des Hohlraums bei vollständig eingesetztem Verschluss nicht mehr verbindet und der Kolben den ersten und den zweiten Teil des Hohlraums gasdicht voneinander trennt.

Durch diesen Aufbau kann der fluide Wirkstoff auch als Quelle für das Gas verwendet werden.

Dabei kann wiederum vorgesehen sein, dass an dem Verschluss zumindest ein Steg und/oder zumindest ein Hohlzylinder angeordnet ist, der sich bei eingesetztem Verschluss in den ersten Teil des Hohlraums erstreckt und beim Verschließen den Kolben in Richtung der zumindest einen Öffnung an dem Überströmkanal vorbei schiebt.

Dadurch sind die beiden Hohlraumteile leicht und automatisch beim Verschließen des zweiten Hohlraums voneinander trennbar.

Gemäß einer Weiterbildung der Erfindung kann vorgesehen sein, dass an dem Verschluss ein Gewinde, vorzugsweise ein Außengewinde angeordnet ist und dass der Hohlraum an der Verschlussöffnung ein Gegengewinde, vorzugsweise ein Innengewinde aufweist, so dass der Verschluss den Kolben bei Einschrauben der Verschlusses in Richtung der zumindest einen Öffnung an dem Überlaufkanal vorbei schiebt.

Hierdurch ist ein kontrolliertes Vortreiben des Kolbens durch den Verschluss (beziehungsweise die Verschlusskappe beziehungsweise die Schraubkappe) möglich. Zudem kann das Gewinde eine Dichtfunktion erfüllen.

Ferner kann erfindungsgemäß vorgesehen sein, dass wenigstens ein Ventilelement zum Gasablass bei Überdruck an dem zweiten Teil des Hohlraums angeordnet ist, vorzugsweise eine Berstscheibe oder ein Überdruckventil an dem zweiten Teil des Hohlraums angeordnet ist, wobei das Ventilelement bevorzugt in dem Kolben angeordnet ist, so dass bei einem ausreichenden Überdruck im zweiten Teil des Hohlraums eine Öffnung zum zweiten Teil des Hohlraums im Kolben entsteht.

Dies erhöht die Sicherheit der Vorrichtung, da so ein unerwünschter Überdruck bei vermieden werden kann.

Erfindungsgemäße Vorrichtungen können sich auch dadurch auszeichnen, dass der zumindest eine Hohlraum, insbesondere die kapillarförmigen Röhren aus synthetischen Polymeren und/oder partialsynthetischen Polymeren besteht oder bestehen.

Diese Werkstoffe sind für erfindungsgemäße Vorrichtungen besonders gut verwendbar und geeignet.

Gemäß einer bevorzugten Weiterbildung der Erfindung kann vorgesehen sein, dass die zumindest eine Öffnung durch einen perforierten Körper geschlossen ist, vorzugsweise durch einen perforierte Gummikörper geschlossen ist, wobei der perforierte Körper im Bereich der zumindest einen Öffnung angeordnet ist.

Hierdurch kann ein vorzeitiges ungewolltes Austreten des fluiden Wirkstoffs aus der Vorrichtung vermieden werden.

Die Aufgaben der Erfindung werden auch gelöst durch ein medizinisches Implantat aufweisend eine solche Vorrichtung, wobei bevorzugt das medizinische Implantat ein Hüftspacer oder ein Kniespacer ist.

Ferner werden die Aufgaben der Erfindung gelöst durch ein Verfahren zur Freisetzung eines fluiden Wirkstoffs aus einem Hohlraum eines medizinischen Implantats, bei dem ein Gas durch eine chemische Reaktion von flüssigem Wasser mit einem Reduktionsmittel erzeugt wird und der entstehende Gasdruck zum Austreiben des fluiden Wirkstoffs aus zumindest einer Öffnung des Hohlraums verwendet wird, wobei der Hohlraum zumindest bereichsweise durch eine Mehrzahl von zumindest bereichsweise parallelen kapillarförmigen Röhren in der Vorrichtung gebildet ist, die sich auf einer Seite bis zu einer Öffnung in der Oberfläche der Vorrichtung erstrecken

Das erfindungsgemäße Verfahren wird vorzugsweise mit einer erfindungsgemäßen Vorrichtung umgesetzt.

Bei dem Verfahren kann vorgesehen sein, dass als Gas Wasserstoff erzeugt wird.

Die Verwendung von Wasserstoff als Gas hat den Vorteil, dass das Wasser der wässrigen Lösungen als Quelle für das Gas verwendet werden kann und so keine zusätzlichen Gasquellen mehr notwendig sind.

Es wird auch vorgeschlagen. dass als Reduktionsmittel eine metallische Oberfläche verwendet wird, bevorzugt eine metallische Oberfläche mit einem negativen Standardpotenzial verwendet wird, besonders bevorzugt Magnesium, Eisen oder Legierungen aufweisend Magnesium und/oder Eisen als metallische Oberfläche verwendet wird, wobei das Gas, insbesondere der Wasserstoff durch eine Korrosion der metallischen Oberfläche erzeugt wird.

Solche Oberflächen korrodieren langsam, so dass eine gleichmäßige und langfristige Freisetzung des Wirkstoffs mit dem Aufbau sichergestellt werden kann.

Dabei kann vorgesehen sein, dass als Reduktionsmittel Magnesium und ein Edelmetall verwendet wird und das Gas durch eine elektrochemische Reaktion erzeugt wird.

Diese Metalle korrodieren mit Wasser ausreichend stark aber auch ausreichend langsam, um eine langfristige gleichmäßige Freisetzung des fluiden Wirkstoffs sicherzustellen. Gleichzeitig sind diese Metalle zur Verwendung im menschlichen Körper aus medizinischer Sicht unbedenklich.

Alternativ kann vorgesehen sein, dass als Reduktionsmittel eine Mischung eines schnell korrodierenden Metalls und eines langsam korrodierenden Metalls verwendet wird.

Hierdurch kann die Korrosion und damit die Fließgeschwindigkeit des fluiden Wirkstoffs noch genauer eingestellt werden.

Schließlich wird mit dem erfindungsgemäßen Verfahren vorgeschlagen, dass das flüssige Wasser, das zur chemischen Reaktion verwendet wird, Teil einer wässrigen Lösung ist, die den fluiden Wirkstoff bildet, wobei bevorzugt eine wässrige, pharmazeutisch aktive Lösung als fluider Wirkstoff verwendet wird, besonders bevorzugt eine wässrige Lösung aufweisend zumindest ein Antibiotikum.

Erfindungsgemäß ist ferner ein Verfahren zur Wirkstofffreisetzung, das dadurch charakterisiert ist, dass eine wässrige Wirkstofflösung aus einem Hohlraum durch Wasserstoff ausgetrieben wird, der durch Korrosion wenigstens eines Metalls oder einer Metalllegierung bei Kontakt mit einer wässrigen Elektrolytlösung entsteht, wobei das wenigstens eine Metall oder die wenigstens eine Metalllegierung zu mindestens in einem Teil des Hohlraums angeordnet ist.

Der Erfindung liegt die überraschende Erkenntnis zugrunde, dass es mit dem erfindungsgemäßen Verfahren und der erfindungsgemäßen Vorrichtung gelingt, Wirkstoffe mit einem aktiven Transportmechanismus über einen Zeitraum bis zu mehreren Wochen kontinuierlich freizusetzen, wobei dieser Transportmechanismus bei Körpertemperatur aktiv ist, ohne dass dazu eine externe oder interne Energiequelle notwendig ist. Die Erfindung beruht auf der überraschenden Erkenntnis, dass Magnesium, Magnesiumlegierungen, Eisen und Eisenlegierungen unter der Einwirkung von Elektrolytlösungen und auch Körperflüssigkeiten, wie Blut, korrodieren, wobei als Nebenprodukt Wasserstoff frei wird und die Produkte dieser chemischen Reaktion (also das Wasserstoffgas) als Treibmittel zum Austragen eines Wirkstoffs aus einem Hohlraum verwendet werden können. Die Korrosionsgeschwindigkeit ist abhängig vom verwendeten Metall beziehungsweise der verwendeten Metalllegierung, vom Oberfläche-Volumen-Verhältnis des Metalls beziehungsweise der Metalllegierung und reicht bei geeignetem Aufbau über einen Zeitraum von mehreren Wochen. Das bedeutet, dass bei dem Korrosionsprozess kontinuierlich Wasserstoff freigesetzt wird, der zum Austreiben des Wirkstoffs aus dem Hohlraum verwendet wird. Bei der Korrosion von Magnesium entsteht Magensiumhydroxid, das ungiftig und völlig unbedenklich ist. Magnesium ist ein natürlicher Bestandteil des humanen Organismus. Eisen korrodiert unter Bildung von Eisenoxiden. Diese sind auch als medizinisch unbedenklich anzusehen. Eisen ist ein natürlicher Bestandteil des humanen Organismus und ist für den Menschen lebenswichtig. Daher sind diese beiden Metalle zur Anwendung in einer erfindungsgemäßen Vorrichtung und mit einem erfindungsgemäßen Verfahren besonders bevorzugt.

Die Grundidee der Erfindung beruht darauf, den bei der Korrosion von Metallen zwangsläufig freigesetzten Wasserstoff als Antrieb zur kontinuierlichen Freisetzung von pharmazeutischen Wirkstofflösungen einzusetzen. Eine Vielzahl von in Wasser löslichen Wirkstoffen liegt in der Salzform vor und bilden nach ihrer Auflösung in Wasser Elektrolytlösungen. Beispielhaft sind dafür die Antibiotika Gentamicinsulfat, Tobramycinsulfat, Clindamycinhydrochlorid und Vancomycinhydrochlorid. Im Fall von nichtsalzförmigen in Wasser löslichen oder zu mindestens suspendierbaren Wirkstoffen kann der Wirkstoff auch in medizinisch übliche Elektrolytlösungen, wie physiologische Kochsalzlösungen oder Ringer-Lösungen gelöst oder suspendiert werden.

Erfindungsgemäß und eine Ausführungsvariante der Erfindung ist eine Vorrichtung zur Wirkstofffreisetzung, die dadurch charakterisiert ist, dass
a) zumindest ein kapillarförmiger Hohlraum, der mindestens eine Öffnung besitzt, in der Vorrichtung angeordnet ist,
b) wobei der Hohlraum mit wässriger Wirkstofflösung zu mindestens teilweise gefüllt ist,
c) wobei die wässrige Wirkstofflösung mindestens an einer Stelle mit einem Metall oder einer Metalllegierung in Kontakt steht,
d) wobei das Metall oder die Metalllegierung durch eine gasdichte Umhüllung eingeschlossen ist und
e) wobei das Metall oder die Metalllegierung flüssigkeitsdurchlässig und gasdurchlässig durch mindestens eine Öffnung der Umhüllung mit dem wenigstens einen kapillarförmigen Hohlraum verbunden ist, so dass der durch Korrosion des Metalls oder der Metalllegierung entstehende Wasserstoff und die wässrige Wirkstofflösung nur durch den mindestens einen kapillarförmigen Hohlkörper austreten können.

Das bedeutet, dass der fluide Wirkstoff, der eine wässrige Wirkstofflösung oder auch eine Wirkstoffsuspension sein kann, wird zuerst in den kapillarförmigen Hohlraum, zum Beispiel durch Aufsaugen, eingebracht. Die Wirkstofflösung kommt in Kontakt mit dem Metall oder der Metalllegierung. Der Korrosionsprozess setzt ein und dabei wird kontinuierlich Wasserstoff freigesetzt. Dieser treibt kontinuierlich die Wirkstofflösung beziehungsweise Wirkstoffsuspension aus dem kapillarförmigen Hohlraum.

Eine vorteilhafte Ausgestaltung der Erfindung besteht darin, dass mindestens eine solche Masse an Metall oder Metalllegierung in der Vorrichtung angeordnet ist, dass durch Korrosion des Metalls oder der Metalllegierung ein Volumen an Wasserstoff freigesetzt wird, das bei Raumtemperatur mindestens 50 Volumenprozent des kapillarförmigen Hohlraums einnimmt.

Für die Freisetzung von ausreichenden Volumina von Wasserstoff genügen kleinste Metallmengen. So setzt 1 mg Magnesium unter Standardbedingungen circa 0,9 ml Wasserstoff frei. Für die kontinuierliche Wirkstofffreisetzung sind Wasserstoff-Volumina von wenigen Millilitern völlig ausreichend. Daher genügen Metalmengen im Milligrammbereich zum Antrieb eines erfindungsgemäßen Freisetzungssystems.

Erfindungsgemäß ist ferner, dass wenigstens zwei kapillarförmige Hohlräume vorhanden sind, wobei Kapillarbündel besonders bevorzugt sind. Die Kapillarbündel beziehungsweise die Bündel von kapillarförmigen Röhren können vorteilhaft in rohrförmigen oder auch beliebig anders geformten Behältern angeordnet sein, wobei mindestens ein Ende der Kapillarbündel für Flüssigkeiten und Gase offen sein muss. Besonders vorteilhaft ist es, wenn die Kapillarbündel in dem Behälter gasdicht mit einem Kunststoff vergossen sind. Die Behälter können einseitig durch Kappen mit Schraubgewinde oder mit durch Rastungen verriegelbaren Kappen verschlossen sein, wobei sich in der Innenseite der Schraubkappe das Metall beziehungsweise die Metalllegierung befindet. Ebenso ist es möglich, zwei Behälter mit Kapillarbündeln zu kombinieren, wobei beide Behälter gasdicht an jeweils einem Ende miteinander gekoppelt werden, wobei das Metall/Metalllegierung am Übergang zwischen den beiden Kapillarbündeln angeordnet ist. Im Rahmen der Erfindung kann es auch vorgesehen sein, dass die Innenseite der kapillarförmigen Hohlräume mit dem Metall oder der Metalllegierungen als Reduktionsmittel beschichtet ist.

Es kann erfindungsgemäß ferner vorgesehen sein, dass die Kapillaren aus synthetischen Polymeren und/oder partialsynthetischen Polymeren bestehen.

Erfindungsgemäß ist weiterhin eine Vorrichtung zur Wirkstofffreisetzung, die dadurch charakterisiert ist, dass
a) ein Hohlkörper in der Vorrichtung angeordnet ist, wobei der Hohlkörper mindestens eine Öffnung aufweist,
b) in einem distalen Teil des Hohlkörpers mindestens ein Überströmkanal an der Innenseite des Hohlraums angeordnet ist,
c) im Hohlraum ein in axialer Richtung des Hohlkörpers verschiebbarer Kolben angeordnet ist,
d) am distalen Ende des Hohlkörpers ein Verschluss angeordnet ist, der an seiner proximalen Seite als Stößel ausgebildet ist, und in Richtung des proximalen Endes des Hohlraums durch Drehung oder Verschiebung bewegt werden kann,
e) wobei der Kolben initial so angeordnet ist, dass der proximale Hohlraum mit dem distalen Hohlraum, der sich zwischen dem Kolben und dem Verschluss befindet, flüssigkeitsdurchlässig verbunden ist, und
f) wobei sich im distalen Hohlraum mindestens ein Körper aus Metall oder einer Metalllegierung befindet, der in Kontakt mit wässriger Lösung unter Freisetzung von Wasserstoff korrodiert.

Als wässrige Lösung kann erfindungsgemäß bevorzugt eine wässrige Elektrolytlösung verwendet werden.

Diese Vorrichtung funktioniert in der Weise, dass die komplett zusammengesetzte Vorrichtung, bei welcher der Kolben zwischen zwei Öffnungen beziehungsweise Enden eines Überströmkanals angeordnet ist, zuerst mit der wässrigen Wirkstofflösung beziehungsweise Wirkstoffsuspension über die mindestens eine Öffnung (Verschlussöffnung) der Vorrichtung befüllt wird. Die beiden Öffnungen des Überströmkanals verbinden den proximalen Hohlraum mit dem distalen Hohlraum. Der Überströmkanal kann als einfache axiale Nut ausgebildet sein. Dabei fließt die wässrige Wirkstofflösung beziehungsweise die wässrige Wirkstoffsuspension durch den Überströmkanal am Kolben vorbei vom proximalen Hohlraum in den distalen Hohlraum. Im distalen Hohlraum befindet sich das Metall oder die Metalllegierung. Die Wirkstofflösung beziehungsweise die Wirkstoffsuspension kommt in Kontakt mit dem Metall oder der Metalllegierung. Dann wird der Verschluss vom Anwender durch Drehung oder durch Verschiebung in Richtung des proximalen Endes der Vorrichtung bewegt. Der Verschluss ist an seinem proximalen Ende als Stößel ausgebildet. Dieser Stößel bewegt den Kolben in proximaler Richtung. Dabei überfährt der Kolben die proximale Öffnung des Überströmkanals. Der distale Hohlraum ist dadurch gasdicht vom proximalen Hohlraum abgeschlossen. Im gasdichten distalen Hohlraum befindet sich zumindest ein kleiner Teil der Wirkstofflösung beziehungsweise der Wirkstoffsuspension und das Metall beziehungsweise die Metalllegierung. Die Korrosion setzt ein. Es bildet sich im distalen Hohlraum kontinuierlich Wasserstoff, der den Kolben in Richtung des proximalen Endes der Vorrichtung bewegt und dadurch die im proximalen Hohlraum befindliche Wirkstofflösung beziehungsweise Wirkstoffsuspension aus der mindestens einen Öffnung (proximalen Öffnung) kontinuierlich herauspresst.

Eine vorteilhafte Ausgestaltungsvariante besteht darin, dass wenigstens ein Öffnungselement zum Gasablass bei Überdruck angeordnet ist, wobei eine Berstscheibe oder ein Überdruckventil bevorzugt sind, die vorzugsweise so im Kolben angeordnet sind, dass bei Überdruck im distalen Hohlraum eine Verbindung zum proximalen Hohlraum hergestellt wird.

Es kann weiterhin vorteilhaft vorgesehen sein, dass der proximale Hohlraum zwei Öffnungen besitzt, die hintereinander axial angeordnet sind, wobei der axiale Abstand der beiden Öffnungen größer ist als die Höhe des Kolbens. Dadurch kann der Kolben am Ende der Auspressbewegung die erste Öffnung überfahren, wobei die restliche Wirkstofflösung beziehungsweise Wirkstoffsuspension durch die zweite Öffnung ausgepresst wird. Wenn die erste Öffnung vom Kolben überfahren ist, dann kann der Wasserstoff entweichen und ein Überdruck in der Vorrichtung kann sicher verhindert werden.

Erfindungsgemäß kann ferner vorgesehen sein, dass mindestens eine solche Masse an Metall oder Metalllegierung angeordnet ist, dass durch Korrosion des Metalls oder der Metallegierung ein Volumen an Wasserstoff freigesetzt wird, das bei Raumtemperatur mindestens 30 Volumenprozent des proximalen Hohlraums einnimmt, bevorzugt 50 Volumenprozent des proximalen Hohlraums, besonders bevorzugt 80 Volumenprozent des proximalen Hohlraums. Der proximale Hohlraum entspricht dem zweiten Teil des Hohlraums.

Die beiden erfindungsgemäßen Wirkstofffreisetzungssysteme können selbst als Hüftspacer, als Kniespacer, als lokaler Antibiotika-Träger und auch als Bestandteil von Hüftspacern, Kniespacern und von lokalen Antibiotika-Trägern verwendet werden.

Daneben können alle sonstigen in Wasser löslichen oder suspendierbaren Wirkstoffe mit den erfindungsgemäßen Wirkstofffreisetzungssystemen, die in beliebiger Gestalt geformt sein können, freigesetzt werden.

Im Folgenden werden Ausführungsbeispiele der Erfindung anhand von fünf schematisch dargestellten Figuren erläutert, ohne jedoch dabei die Erfindung zu beschränken. Dabei zeigt:
- Figur 1:: eine schematische Querschnittansicht einer erfindungsgemäßen Vorrichtung und
- Figur 2:: eine schematische Querschnittansicht einer alternativen erfindungsgemäßen Vorrichtung.

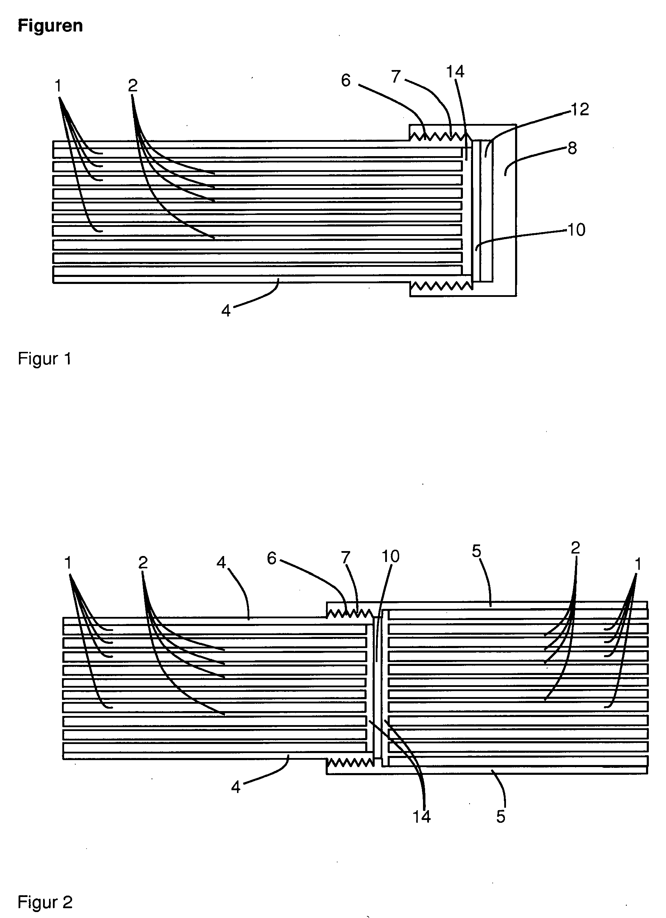

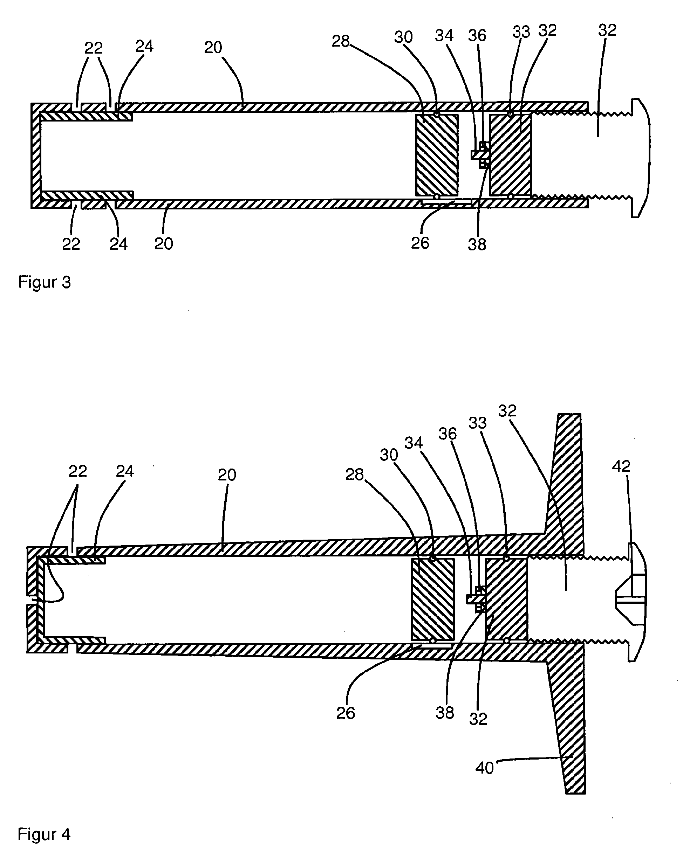

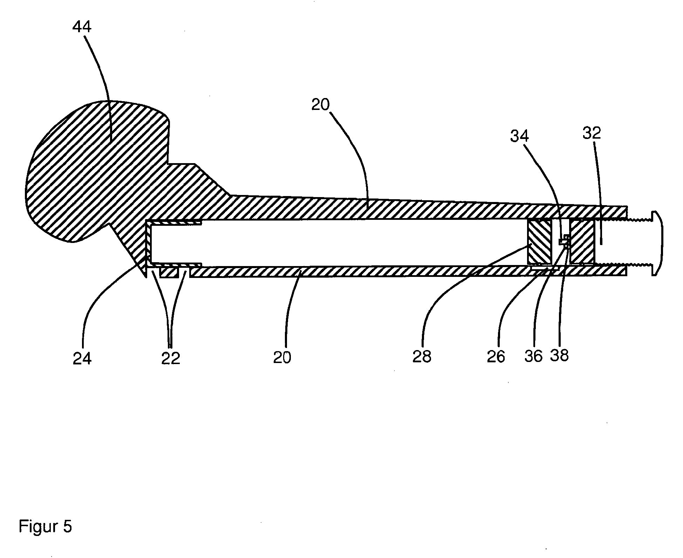

Figur 1 zeigt eine schematische Querschnittansicht einer erfindungsgemäßen Vorrichtung. Die Vorrichtung beinhaltet einen Grundkörper 1 aus einem Polymer, der eine Vielzahl von Kapillaren 2 umfasst. Die Kapillaren 2 können aus einem synthetischen oder einem partialsynthetischen Polymer gefertigt sein, die entweder in den Grundkörper 1 eingegossen sind oder aus dem gleichen Material wie der Grundkörper 1 gefertigt sind und in dem oder mit dem Grundkörper 1 erzeugt wurden. Der Grundkörper 1 ist in einem Gehäuse 4 aus einem biokompatiblen Material angeordnet.

An einem Ende des Gehäuses 4 ist ein Außengewinde 6 vorgesehen, auf das ein Innengewinde 7 einer Kappe 8 aufgeschraubt ist. Die Kappe 8 besteht ebenfalls aus einem biokompatiblen Material. Im Deckel der Kappe 8 ist eine Metallplatte 10 aus Magnesium und/oder Eisen angeordnet, die über eine elastische Verbindung 12 mit dem Deckel der Kappe 8 verbunden ist. Die Gewinde 6, 7 sind derart aufgebaut, dass bei einem vollständigen Aufschrauben der Kappe 8 auf das Gehäuse 4 der Anschlag so gewählt ist, dass sich zwischen dem Grundkörper 1 und der Kappe 8 ein Innenraum 14 bildet. Statt der elastischen Verbindung 12 kann sich an dieser Stelle auch der Innenraum 14 fortsetzen, so dass die Metallplatte 10 weitgehend vom Innenraum 14, beziehungsweise von dessen Inhalt umschlossen ist. Der Innenraum 14 und die Kapillaren 2 bilden in der Vorrichtung einen Hohlraum 2, 14. In diesem Hohlraum 2, 14 in der Vorrichtung, also in den Kapillaren 2 und dem Innenraum 14 ist eine wässrige Antibiotika-Lösung enthalten.

Figur 2 zeigt eine schematische Querschnittansicht eines ähnlichen Aufbaus. Der Aufbau umfasst zwei Grundkörper 1 mit Kapillaren 2, die in einem ersten Gehäuse 4 und in einem zweiten Gehäuse 5 angeordnet sind. Das erste Gehäuse 4 weist ein Außengewinde 6 und das zweite Gehäuse 5 ein Innengewinde 7 auf und die beiden Gehäuse 4, 5 sind miteinander verschraubt. Zwischen den beiden Gehäusen 4, 5 ist eine Metallplatte 10 aus Magnesium und/oder Eisen oder einer Legierung daraus angeordnet, die Teil eines der beiden Gehäuse 4, 5 sein kann aber auch eine separate Metallplatte 10 sein kann, die zwischen den Gehäusen 4, 5 angeordnet wird, bevor diese zusammengeschraubt werden. Zwischen den Grundkörpern 1 und der Metallplatte 10 bilden sich Innenräume 14 in jedem Gehäuse 4, 5. Bevorzugt schließt die Metallplatte 10 dicht mit den Wänden zumindest eines der Gehäuse 4, 5, so dass die Innenräume 14 voneinander getrennt sind. Es ist jedoch auch möglich, dass die Innenräume 14 miteinander durch die Metallplatte 10 oder durch Kanäle an oder in den Gehäusen 4, 5 miteinander verbunden sind. Dann wird in jedem Innenraum 14 der gleiche Gasdruck aufgebaut (siehe hierzu die Erläuterung in der folgenden Beschreibung).

Die Kapillaren 2 und die Innenräume 14 bilden Hohlräume 2, 14 in der Vorrichtung, die mit einer wässrigen Lösung zumindest einer pharmazeutisch aktiven Substanz gefüllt sind. In beiden Ausführungsbeispielen nach den Figuren 1 und 2 wird dann das gleiche erfindungsgemäße Verfahren realisiert:
Das Wasser in der wässrigen Antibiotika-Lösung beziehungsweise der wässrigen Lösung der pharmazeutisch aktiven Substanz reagiert chemisch an der Metalloberfläche der Metallplatte mit dem Magnesium und/oder dem Eisen, wobei das Metall oder die Metalle korrodiert werden, wodurch Metalloxide entstehen, und gasförmiger Wasserstoff entsteht. Das entstehende Wasserstoffgas hat unter Normalbedingungen ein wesentlich größeres Volumen, so dass es sich im Innenraum 14 ausdehnt und dabei die wässrige Lösung aus den Kapillaren 2 aus der Vorrichtung herausdrückt. Die chemische Reaktion der Korrosion der Metallplatte 10 läuft dabei so langsam ab, dass die wässrige Lösung nur langsam aus den Kapillaren 2 herausgedrückt wird und daher die Vorrichtung die Wirkstoffe über einen langen Zeitraum gleichmäßig abgibt. Die Dauer und die Geschwindigkeit der Wasserstoffentstehung und damit der Wirkstofffreigabe der Vorrichtung können durch die Wahl des Reduktionsmittels, das heißt, durch die Wahl der Metalle der Legierung und deren Oberflächenbeschaffenheit eingestellt werden.

Im Folgenden werden weitere erfindungsgemäße Modifikationen anhand der Figuren 1 und 2 diskutiert. Die Kapillarbündel 2 beziehungsweise die Bündel von kapillarförmigen Röhren 2 sind beispielsweise in rohrförmigen Behältern 4, 5 angeordnet, wobei mindestens ein Ende der Kapillarbündel 2 für Flüssigkeiten und Gase offen ist. Besonders günstig ist es, wenn die Kapillarbündel 2 in dem Behälter 4, 5 gasdicht mit einem Kunststoff vergossen sind. Die Behälter 4 können einseitig durch Kappen 8 mit Schraubgewinde 7 oder mit durch Rastungen verriegelbare Kappen 8 verschlossen sein, wobei sich in der Innenseite der Schraubkappe 8 beziehungsweise der Verschlusskappe 8 das Metall beziehungsweise die Metalllegierung 10 befindet, die als Reduktionsmittel verwendet werden. Ebenso ist es möglich, zwei Behälter 4, 5 mit Kapillarbündeln 2 zu kombinieren, wobei beide Behälter 4, 5 gasdicht an jeweils einem Ende miteinander gekoppelt werden, wobei das Metall beziehungsweise die Metallegierung 10 am Übergang zwischen den beiden Kapillarbündeln 2 angeordnet ist. Im Rahmen der Erfindung kann auch vorgesehen sein, dass die Innenseite der kapillarförmigen Hohlräume 2 mit Metall oder mit Metalllegierungen beschichtet ist. Die chemische Reaktion, bei der der Wasserstoff als Treibmittel zum Austreiben der wässrigen Lösung erzeugt wird, muss also nicht nur im Innenraum 14 oder den Innenräumen 14 ablaufen sondern kann auch in den Kapillaren 2 selbst stattfinden.

### Bezugszeichenliste

- 1: Grundkörper
- 2: Kapillare
- 4, 5: Gehäuse
- 6: Außengewinde
- 7: Innengewinde
- 8: Kappe
- 10: Metallplatte
- 12: Elastische Verbindung
- 14: Innenraum
- 20: Hohlkörper
- 22: Öffnung
- 24: Perforierte Gummihülse
- 26: Überströmkanal
- 28: Kolben
- 30, 33: O-Ring / Dichtung
- 32: Schraubkappe / Verschluss
- 34: Stößel
- 36: Schwamm
- 38: Magnesium- / Eisenplättchen
- 40: Platte
- 42: Kreuzschlitz
- 44: Kopf eines Femurspacers

## Patentansprüche

1. Vorrichtung zur Freisetzung eines fluiden Wirkstoffs aufweisend wenigstens einen Hohlraum (2, 14), in dem der fluide Wirkstoff enthalten ist, wobei in der Vorrichtung Wasser und ein Reduktionsmittel (10, 38) enthalten sind, die miteinander in Verbindung stehen oder miteinander in Verbindung bringbar sind, so dass durch eine chemische Reaktion des Wassers mit dem Reduktionsmittel (10, 38) in der Vorrichtung ein Gas entsteht und das Gas derart mit dem fluiden Wirkstoff in Wirkverbindung steht, das der entstehende Gasdruck den fluiden Wirkstoff aus zumindest einer Öffnung (22) des Hohlraums (2, 14) aus der Vorrichtung herausdrückt, wobei der Hohlraum (2, 14) zumindest bereichsweise durch eine Mehrzahl von zumindest bereichsweise parallelen kapillarförmigen Röhren (2) in der Vorrichtung gebildet ist, die sich auf einer Seite bis zu einer Öffnung in der Oberfläche der Vorrichtung erstrecken.

2. Vorrichtung nach Anspruch 1, **dadurch gekennzeichnet, dass**
das entstehende Gas Wasserstoff ist.

3. Vorrichtung nach Anspruch 1 oder 2, **dadurch gekennzeichnet, dass** das Wasser für die chemische Reaktion ein Teil einer flüssigen wässrigen Lösung ist, die den fluiden Wirkstoff bildet, wobei bevorzugt der fluide Wirkstoff eine wässrige Lösung aufweisend zumindest eine pharmazeutisch aktive Substanz ist, besonders bevorzugt eine wässrige Lösung aufweisend zumindest ein Antibiotikum.

4. Vorrichtung nach einem der vorangehenden Ansprüche, **dadurch gekennzeichnet, dass** das Reduktionsmittel (10, 38) eine metallische Oberfläche (10, 38) ist, wobei bevorzugt die metallische Oberfläche (10, 38) ein negatives Standardpotential aufweist, besonders bevorzugt die metallische Oberfläche (10, 38) Magnesium, Eisen oder Legierungen aufweisend Magnesium und/oder Eisen beinhaltet oder daraus besteht.

5. Vorrichtung nach Anspruch 4, **dadurch gekennzeichnet, dass**
das Reduktionsmittel (10, 38) Magnesium und ein Edelmetall ist, die eine elektrochemische Zelle bilden, oder das Reduktionsmittel (10, 38) eine Mischung eines schnell korrodierenden Metalls und eines langsam korrodierenden Metalls ist.

6. Vorrichtung nach einem der vorangehenden Ansprüche, **dadurch gekennzeichnet, dass** mindestens eine solche Masse an Reduktionsmittel (10, 38) und/oder an Wasser in der Vorrichtung angeordnet ist, dass eine solche Menge an Gas entsteht, die zumindest 30% des Volumens des Hohlraums (2, 14) einnimmt, bevorzugt zumindest 50% des Volumens des Hohlraums (2, 14), besonders bevorzugt zumindest 80% des Volumens des Hohlraums (2, 14).

7. Vorrichtung nach einem der vorangehenden Ansprüche, **dadurch gekennzeichnet, dass** das Reduktionsmittel (10, 38) an dem zu der jeweiligen Öffnung gegenüberliegenden Ende der kapillarförmigen Röhren angeordnet ist, wobei das Reduktionsmittel (10, 38) in dem Hohlraum (2, 14) angeordnet ist und nur über die Öffnung der Röhre (2) mit der Oberfläche der Vorrichtung verbunden ist, wobei vorzugweise der fluide Wirkstoff als wässrige Lösung mit dem Reduktionsmittel (10, 38) in Kontakt steht.

8. Vorrichtung nach einem der Ansprüche 1 bis 6, **dadurch gekennzeichnet, dass** in dem Hohlraum (2, 14) ein in Richtung der zumindest einen Öffnung (22) des Hohlraums (2, 14) verschiebbarer Kolben (28) angeordnet ist und der Kolben (28) den Hohlraum (2, 14) in einen ersten Teil, der die zumindest eine Öffnung (22) aufweist, und einen zweiten geschlossenen oder verschließbaren Teil unterteilt, wobei in dem ersten Teil der fluide Wirkstoff angeordnet ist und in dem zweiten Teil das Reduktionsmittel (10, 38) und Wasser angeordnet sind, so dass der entstehende Gasdruck in dem geschlossenen, zweiten Teil des Hohlraums (2, 14) den Kolben (28) in Richtung der zumindest einen Öffnung (22) treibt und dadurch den fluiden Wirkstoff aus dem ersten Teil des Hohlraums (2, 14) durch die zumindest eine Öffnung (22) aus der Vorrichtung herausdrückt.

9. Vorrichtung nach Anspruch 8, **dadurch gekennzeichnet, dass**
in der Innenwand des Hohlraums (2, 14) ein Überströmkanal (26) angeordnet ist, der in einer Ausgangsstellung des Kolbens (28) den ersten Teil und den zweiten Teil des Hohlraums (2, 14) verbindet und der zweite Teil des Hohlraums (2, 14) eine Verschlussöffnung aufweist, die durch einen Verschluss (32) verschließbar oder geschlossen ist, so dass der Hohlraum (2, 14) in beiden Teilen mit der gleichen wässrigen Lösung, die den fluiden Wirkstoff bildet und das flüssige Wasser für die chemische Reaktion bereitstellt, durch die Verschlussöffnung befüllbar ist, wobei der Verschluss (32) derart aufgebaut ist, dass er beim Verschließen der Verschlussöffnung den Kolben (28) in dem Hohlraum (2, 14) in Richtung der zumindest einen Öffnung (22) an dem Überströmkanal (26) vorbei schiebt, so dass der Überströmkanal (26) den ersten und den zweiten Teil des Hohlraums (2, 14) bei vollständig eingesetztem Verschluss (32) nicht mehr verbindet und der Kolben (28) den ersten und den zweiten Teil des Hohlraums (2, 14) gasdicht voneinander trennt.

10. Vorrichtung nach Anspruch 9, **dadurch gekennzeichnet, dass**
an dem Verschluss (32) zumindest ein Steg (34) und/oder zumindest ein Hohlzylinder angeordnet ist, der sich bei eingesetztem Verschluss (32) in den ersten Teil des Hohlraums (2, 14) erstreckt und beim Verschließen den Kolben (28) in Richtung der zumindest einen Öffnung (22) an dem Überströmkanal (26) vorbei schiebt.

11. Vorrichtung nach Anspruch 9 oder 10, **dadurch gekennzeichnet, dass**
an dem Verschluss (32) ein Gewinde, vorzugsweise ein Außengewinde angeordnet ist und dass der Hohlraum (2, 14) an der Verschlussöffnung ein Gegengewinde, vorzugsweise ein Innengewinde aufweist, so dass der Verschluss (32) den Kolben (28) bei Einschrauben der Verschlusses (32) in Richtung der zumindest einen Öffnung (22) an dem Überlaufkanal (26) vorbei schiebt.

12. Vorrichtung nach einem der Ansprüche 8 bis 11, **dadurch gekennzeichnet, dass** wenigstens ein Ventilelement zum Gasablass bei Überdruck an dem zweiten Teil des Hohlraums (2, 14) angeordnet ist, vorzugsweise eine Berstscheibe oder ein Überdruckventil an dem zweiten Teil des Hohlraums (2, 14) angeordnet ist, wobei das Ventilelement bevorzugt in dem Kolben (28) angeordnet ist, so dass bei einem ausreichenden Überdruck im zweiten Teil des Hohlraums (2, 14) eine Öffnung zum zweiten Teil des Hohlraums (2, 14) im Kolben (28) entsteht.

13. Vorrichtung nach einem der vorangehenden Ansprüche, **dadurch gekennzeichnet, dass** der zumindest eine Hohlraum (2, 14), insbesondere die kapillarförmigen Röhren (2) aus synthetischen Polymeren und/oder partialsynthetischen Polymeren besteht oder bestehen.

14. Vorrichtung nach einem der vorangehenden Ansprüche, **dadurch gekennzeichnet, dass** die zumindest eine Öffnung (22) durch einen perforierten Körper (24) geschlossen ist, vorzugsweise durch einen perforierte Gummikörper (24) geschlossen ist, wobei der perforierte Körper (24) im Bereich der zumindest einen Öffnung (22) angeordnet ist.

15. Medizinisches Implantat aufweisend eine Vorrichtung nach einem der vorangehenden Ansprüche, wobei bevorzugt das medizinische Implantat ein Hüftspacer oder ein Kniespacer ist.

16. Verfahren zur Freisetzung eines fluiden Wirkstoffs aus einem Hohlraum (2, 14) eines medizinischen Implantats, bei dem ein Gas durch eine chemische Reaktion von flüssigem Wasser mit einem Reduktionsmittel (10, 38) erzeugt wird und der entstehende Gasdruck zum Austreiben des fluiden Wirkstoffs aus zumindest einer Öffnung des Hohlraums (2, 14) verwendet wird, wobei der Hohlraum (2, 14) zumindest bereichsweise durch eine Mehrzahl von zumindest bereichsweise parallelen kapillarförmigen Röhren (2) in der Vorrichtung gebildet ist, die sich auf einer Seite bis zu einer Öffnung in der Oberfläche der Vorrichtung erstrecken.

17. Verfahren nach Anspruch 16, **dadurch gekennzeichnet, dass**
als Gas Wasserstoff erzeugt wird.

18. Verfahren nach Anspruch 16 oder 17, **dadurch gekennzeichnet, dass**
als Reduktionsmittel (10, 38) eine metallische Oberfläche (10, 38) verwendet wird, bevorzugt eine metallische Oberfläche (10, 38) mit einem negativen Standardpotenzial verwendet wird, besonders bevorzugt Magnesium, Eisen oder Legierungen aufweisend Magnesium und/oder Eisen als metallische Oberfläche (10, 38) verwendet wird, wobei das Gas, insbesondere der Wasserstoff durch eine Korrosion der metallischen Oberfläche (10, 38) erzeugt wird.

19. Verfahren nach Anspruch 18, **dadurch gekennzeichnet, dass**
als Reduktionsmittel (10, 38) Magnesium und ein Edelmetall verwendet wird und das Gas durch eine elektrochemische Reaktion erzeugt wird.

20. Verfahren nach Anspruch 18, **dadurch gekennzeichnet, dass**
als Reduktionsmittel (10, 38) eine Mischung eines schnell korrodierenden Metalls und eines langsam korrodierenden Metalls verwendet wird.

21. Verfahren nach einem der Ansprüche 16 bis 20, **dadurch gekennzeichnet, dass**
das flüssige Wasser, das zur chemischen Reaktion verwendet wird, Teil einer wässrigen Lösung ist, die den fluiden Wirkstoff bildet, wobei bevorzugt eine wässrige, pharmazeutisch aktive Lösung als fluider Wirkstoff verwendet wird, besonders bevorzugt eine wässrige Lösung aufweisend zumindest ein Antibiotikum.

## Claims

1. Device for releasing a fluid active substance, comprising at least one hollow space (2, 14) containing the fluid active substance, whereby the device contains water and a reducing agent (10, 38), which are or can be made to be in communication such that a chemical reaction of the water and the reducing agent (10, 38) generates a gas in the device and the gas effectively communicates with the fluid active substance such that the gas pressure thus generated presses the fluid active substance out of at least one opening (22) of the hollow space (2, 14) out of the device, whereby the hollow space (2, 14) in the device is formed, at least in regions thereof, by a plurality of capillary-shaped tubes (2), which are parallel, at least in regions thereof, and extend on one side up to an opening in the surface of the device.

2. Device according to claim 1, **characterised in that** the gas generated is hydrogen.

3. Device according to claim 1 or 2, **characterised in that**
the water for the chemical reaction is part of a liquid aqueous solution that forms the fluid active substance, whereby the fluid active substance preferably is an aqueous solution comprising at least one pharmaceutically active substance, particularly preferably an aqueous solution comprising at least one antibiotic.

4. Device according to any one of the preceding claims, **characterised in that** the reducing agent (10, 38) is a metallic surface (10, 38), whereby the metallic surface (10, 38) preferably has a negative standard potential, whereby the metallic surface (10, 38) particularly preferably contains or consists of magnesium, iron or alloys comprising magnesium and/or iron.

5. Device according to claim 4, **characterised in that**
the reducing agent (10, 38) is magnesium and a precious metal forming an electrochemical cell or the reducing agent (10, 38) is a mixture of a quickly corroding metal and a slowly corroding metal.

6. Device according to any one of the preceding claims, **characterised in that** at least a sufficient mass of reducing agent (10, 38) and/or water to produce an amount of gas that takes up at least 30% of the volume of the hollow space (2, 14), preferably at least 50% of the volume of the hollow space (2, 14), particularly preferably at least 80% of the volume of the hollow space (2, 14), is arranged in the device.

7. Device according to any one of the preceding claims, **characterised in that** the reducing agent (10, 38) is arranged on the end of the capillary-shaped tubes that is opposite from the corresponding opening, whereby the reducing agent (10, 38) is arranged in the hollow space and is connected to the surface of the device solely through the opening of the tube (2), whereby the fluid active substance preferably contacts the reducing agent (10, 38) as an aqueous solution.

8. Device according to any one of the claims 1 to 6, **characterised in that** a plunger (28) is arranged in the hollow space (2, 14) that can be shifted towards the at least one opening (22) of the hollow space (2, 14), and the plunger (28) subdivides the hollow space (2, 14) into a first part comprising the at least one opening (22) and a second part that is or can be closed, whereby the fluid active substance is arranged in the first part and the reducing agent (10, 38) and water are arranged in the second part such that the gas pressure generated in the closed second part of the hollow space (2, 14) drives the plunger (28) towards the at least one opening (22) and thus presses the fluid active substance out of the first part of the hollow space (2, 14) through the at least one opening (22) out of the device.

9. Device according to claim 8, **characterised in that**
an overflow channel (26) is arranged in the internal wall of the hollow space (2, 14), which, in a starting position of the plunger (28), connects the first part and the second part of the hollow space (2, 14), and the second part of the hollow space (2, 14) comprises a closure opening, which can be or is closed by means of a closure (32) such that both parts of the hollow space (2, 14) can be filled through the closure opening with the same aqueous solution, which forms the active substance and provides the liquid water for the chemical reaction, whereby the closure (32) is designed such that it pushes the plunger (28) in the hollow space (2, 14) towards the at least one opening (22) past the overflow channel (26) upon the closure opening being closed such that the overflow channel (26) no longer connects the first part and the second part of the hollow space (2, 14) when the closure (32) is fully inserted and the plunger (28) separates the first and the second part of the hollow space (2, 14) from each other in a gas-tight manner.

10. Device according to claim 9, **characterised in that**
at least one fin (34) and/or at least one hollow cylinder is/are arranged on the closure (32) and extend into the first part of the hollow space (2, 14) when the closure (32) is inserted and pushes the plunger (28) towards the at least one opening (22) past the overflow channel (26) upon closing.

11. Device according to claim 9 or 10, **characterised in that**
a thread, preferably an external thread, is arranged on the closure (32), and the hollow space (2, 14) comprises a counter-thread, preferably an internal thread, on the closure opening, such that the closure (32) pushes the plunger (28) towards the at least one opening (22) past the overflow channel (26) when the closure (32) is being screwed in.

12. Device according to any one of the claims 8 to 11, **characterised in that** at least one valve element for gas discharge in the presence of an overpressure is arranged on the second part of the hollow space (2, 14), preferably a burst disc or an overpressure valve is arranged on the second part of the hollow space (2, 14), whereby the valve element preferably is arranged in the plunger (28) such that an opening towards the second part of the hollow space (2, 14) is produced in the plunger (28) in the presence of a sufficient overpressure.

13. Device according to any one of the preceding claims, **characterised in that** the at least one hollow space (2, 14), in particular the capillary-shaped tubes (2), consist(s) of synthetic polymers and/or partially-synthetic polymers.

14. Device according to any one of the preceding claims, **characterised in that** the at least one opening (22) is closed by means of a perforated body (24), preferably is closed by means of a perforated rubber body (24), whereby the perforated body (24) is arranged in the region of the at least one opening (22).

15. Medical implant comprising a device according to any one of the preceding claims, whereby the medical implant preferably is a hip spacer or a knee spacer.

16. Method for releasing a fluid active substance from a hollow space (2, 14) of a medical implant, in which a chemical reaction of liquid water and a reducing agent (10, 38) generates a gas and the gas pressure thus generated is used to expel the fluid active substance out of at least one opening of the hollow space (2, 14), whereby the hollow space (2, 14) is formed in the device, at least in regions thereof, by a plurality of capillary-shaped tubes (2), which are parallel, at least in regions thereof, and extend on one side up to an opening in the surface of the device.

17. Method according to claim 16, **characterised in that** the gas generated is hydrogen.

18. Method according to claim 16 or 17, **characterised in that**
a metallic surface (10, 38) is used as reducing agent (10, 38), preferably a metallic surface (10, 38) having a negative standard potential is used, particularly preferably magnesium, iron or alloys comprising magnesium and/or iron is used as metallic surface (10, 38), whereby the gas, in particular the hydrogen, is produced through corrosion of the metallic surface (10, 38).

19. Method according to claim 18, **characterised in that**
magnesium and a precious metal are used as reducing agent (10, 38) and the gas is produced through an electrochemical reaction.

20. Method according to claim 18, **characterised in that**
a mixture of a quickly corroding metal and a slowly corroding metal is used as reducing agent (10, 38).

21. Method according to any one of the claims 16 to 20, **characterised in that** the liquid water used for the chemical reaction is part of an aqueous solution that forms the fluid active substance, whereby it is preferred to use an aqueous, pharmaceutically active solution as fluid active substance, particularly preferably an aqueous solution comprising at least one antibiotic.

## Revendications

1. Dispositif de libération d'une substance active fluide présentant au moins un espace creux (2, 14) dans lequel la substance active fluide est contenue, dans lequel de l'eau et un réducteur (10, 38) sont contenus dans le dispositif, lesquels sont en liaison l'un avec l'autre ou peuvent être amenés en liaison l'un avec l'autre de sorte qu'un gaz apparaît par une réaction chimique de l'eau avec le réducteur (10, 38) dans le dispositif et le gaz est en liaison active avec la substance active fluide de telle sorte que la pression gazeuse résultante extrait par pression la substance active fluide d'au moins une ouverture (22) de l'espace creux (2, 14) du dispositif, dans lequel l'espace creux (2, 14) est formé au moins par région par une pluralité de tuyaux capillaires (2) parallèles au moins par région dans le dispositif qui s'étendent d'un côté jusqu'à une ouverture dans la surface du dispositif.

2. Dispositif selon la revendication 1, **caractérisé en ce que**
le gaz résultant est de l'hydrogène.

3. Dispositif selon la revendication 1 ou 2, **caractérisé en ce que**
l'eau pour la réaction chimique est une partie d'une solution aqueuse liquide qui forme la substance active fluide, dans lequel la substance active fluide est de préférence une solution aqueuse présentant au moins une substance pharmaceutiquement active, de manière particulièrement préférée une solution aqueuse présentant au moins un antibiotique.

4. Dispositif selon une des revendications précédentes, **caractérisé en ce que**
le réducteur (10, 38) est une surface métallique (10, 38), dans lequel la surface métallique (10, 38) présente de préférence un potentiel standard négatif, de manière particulièrement préférée la surface métallique (10, 38) contient du magnésium, fer ou des alliages présentant du magnésium et/ou fer ou se compose de ceux-ci.

5. Dispositif selon la revendication 4, **caractérisé en ce que**
le réducteur (10, 38) est du magnésium et un métal noble qui forment une cellule électrochimique, ou le réducteur (10, 38) est un mélange d'un métal rapidement corrosif et d'un métal lentement corrosif.

6. Dispositif selon une des revendications précédentes, **caractérisé en ce que** au moins une telle masse de réducteur (10, 38) et/ou d'eau est disposée dans le dispositif qu'une telle quantité de gaz apparaît, laquelle prend au moins 30 % du volume de l'espace creux (2, 14), de préférence au moins 50 % du volume de l'espace creux (2, 14), de manière particulièrement préférée au moins 80 % du volume de l'espace creux (2, 14).

7. Dispositif selon une des revendications précédentes, **caractérisé en ce que**
le réducteur (10, 38) est disposé à l'extrémité opposée à l'ouverture respective des tuyaux capillaires, dans lequel le réducteur (10, 38) est disposé dans l'espace creux (2, 14) et est relié à la surface du dispositif uniquement par le biais de l'ouverture des tuyaux (2), dans lequel la substance active fluide est de préférence en contact en tant que solution aqueuse avec le réducteur (10, 38).

8. Dispositif selon une des revendications 1 à 6, **caractérisé en ce que**
un piston (28) pouvant coulisser en direction de l'au moins une ouverture (22) de l'espace creux (2, 14) est disposé dans l'espace creux (2,14), et le piston (28) divise l'espace creux (2, 14) en une première partie qui présente l'au moins une ouverture (22) et une seconde partie fermée ou pouvant être fermée, dans lequel la substance active fluide est disposée dans la première partie, et le réducteur (10, 38) et l'eau sont disposés dans la seconde partie de sorte que la pression gazeuse résultante dans la seconde partie fermée de l'espace creux (2, 14) entraîne le piston (28) en direction de l'au moins une ouverture (22) et extrait de ce fait par pression la substance active fluide de la première partie de l'espace creux (2, 14) à travers l'au moins une ouverture (22) du dispositif.

9. Dispositif selon la revendication 8, **caractérisé en ce que**
un canal de décharge (26) est disposé dans la paroi interne de l'espace creux (2, 14), lequel relie dans une position de départ du piston (28) la première partie et la seconde partie de l'espace creux (2, 14), et la seconde partie de l'espace creux (2, 14) présente une ouverture de fermeture qui peut être fermée ou est fermée par une fermeture (22) de sorte que l'espace creux (2, 14) peut être rempli à travers l'ouverture de fermeture dans les deux parties de la même solution aqueuse qui forme la substance active fluide et met à disposition l'eau liquide pour la réaction chimique, dans lequel la fermeture (32) est construite de telle sorte qu'elle pousse le piston (28) dans l'espace creux (2, 14) en direction de l'au moins une ouverture (22) devant le canal de décharge (26) lors de la fermeture de l'ouverture de fermeture de sorte que le canal de décharge (26) ne relie plus la première et la seconde partie de l'espace creux (2, 14) en cas de fermeture entièrement insérée (32), et le piston (28) sépare la première et la seconde partie de l'espace creux (2, 14) l'une de l'autre de manière étanche au gaz.

10. Dispositif selon la revendication 9, **caractérisé en ce que**
au moins un montant (34) et/ou au moins un cylindre creux est disposé sur l'ouverture (32), lequel s'étend en cas de fermeture insérée (32) dans la première partie de l'espace creux (2, 14) et pousse le piston (28) en direction de l'au moins une ouverture (22) devant le canal de décharge (26) lors de la fermeture.

11. Dispositif selon la revendication 9 ou 10, **caractérisé en ce que**
un filetage, de préférence un filetage externe, est disposé sur la fermeture (32) et que l'espace creux (2, 14) présente sur l'ouverture de fermeture un filetage conjugué, de préférence un filetage interne, de sorte que la fermeture (32) pousse le piston (28) lors du vissage de la fermeture (32) en direction de l'au moins une ouverture (22) devant le canal de décharge (26).

12. Dispositif selon une des revendications 8 à 11, **caractérisé en ce que**
au moins un élément de soupape pour la purge de gaz en cas de surpression est disposé sur la seconde partie de l'espace creux (2, 14), de préférence une plaque de rupture ou une soupape de sûreté est disposée sur la seconde partie de l'espace creux (2, 14), dans lequel l'élément de soupape est de préférence disposé dans le piston (28) de sorte qu'en cas de surpression suffisante dans la seconde partie de l'espace creux (2, 14), une ouverture vers la seconde partie de l'espace creux (2, 14) apparaît dans le piston (28).

13. Dispositif selon une des revendications précédentes, **caractérisé en ce que** l'au moins un espace creux (2, 14), notamment les tuyaux capillaires (2), se compose ou se composent de polymères synthétiques et/ou de polymères partiellement synthétiques.

14. Dispositif selon une des revendications précédentes, **caractérisé en ce que** l'au moins une ouverture (22) est fermée par un corps perforé (24), est de préférence fermée par un corps en caoutchouc perforé (24), dans lequel le corps perforé (24) est disposé dans la région de l'au moins une ouverture (22).

15. Implant médical présentant un dispositif selon une des revendications précédentes, dans lequel l'implant médical est de préférence un écarteur de hanche ou un écarteur de genou.

16. Procédé de libération d'une substance active fluide d'un espace creux (2, 14) d'un implant médical, dans lequel un gaz est généré par une réaction chimique d'eau liquide avec un réducteur (10, 38) et la pression gazeuse résultante est utilisée pour expulser la substance active fluide d'au moins une ouverture de l'espace creux (2, 14), dans lequel l'espace creux (2, 14) est formé au moins par région par une pluralité de tuyaux capillaires (2) parallèles au moins par région dans le dispositif qui s'étendent d'un côté jusqu'à une ouverture dans la surface du dispositif.

17. Procédé selon la revendication 16, **caractérisé en ce que**
de l'hydrogène est généré comme gaz.

18. Procédé selon la revendication 16 ou 17, **caractérisé en ce que**
une surface métallique (10, 38) est utilisée comme réducteur (10, 38), une surface métallique (10, 38) avec un potentiel standard négatif est de préférence utilisée, de manière particulièrement préférée du magnésium, fer ou des alliages présentant du magnésium et/ou fer sont utilisés comme surface métallique (10, 38), dans lequel le gaz, notamment l'hydrogène, est généré par une corrosion de la surface métallique (10, 38).

19. Procédé selon la revendication 18, **caractérisé en ce que**
du magnésium et un métal noble sont utilisés comme réducteur (10, 38) et le gaz est généré par une réaction électrochimique.

20. Procédé selon la revendication 18, **caractérisé en ce que**
un mélange d'un métal rapidement corrosif et d'un métal lentement corrosif est utilisé comme réducteur (10, 38).

21. Procédé selon une des revendications 16 à 20, **caractérisé en ce que** l'eau liquide qui est utilisée pour la réaction chimique est une partie d'une solution aqueuse qui forme la substance active fluide, dans lequel une solution aqueuse pharmaceutiquement active est de préférence utilisée comme substance active fluide, de manière particulièrement préférée une solution aqueuse présentant au moins un antibiotique.
